# EUROPEAN PATENT SPECIFICATION

(11) **EP 4 215 239 B1**
(45) Date of publication and mention of the grant of the patent: **19.11.2025**
(21) Application number: 22460068.4
(22) Date of filing: 13.12.2022
(51) Int. Cl.: A61N 1/36

(54) **SYSTEM FOR IMPROVING COGNITIVE FUNCTIONS OF THE HUMAN BRAIN**
SYSTEM ZUR VERBESSERUNG KOGNITIVER FUNKTIONEN DES MENSCHLICHEN GEHIRNS
SYSTÈME POUR AMÉLIORER LES FONCTIONS COGNITIVES DU CERVEAU HUMAIN

(30) Priority: 24.01.2022 PL 44020822
(43) Date of publication of application: 26.07.2023
(73) Proprietor: Politechnika Gdanska, 80-233 Gdansk (PL)
(72) Inventor: Lech, Michal, 81-402 Gdynia (PL); Kucewicz, Michal, 80-881 Gdansk (PL); Topcu, Cagdas, 80-226 Gdansk (PL); Czyzewski, Andrzej, 81-577 Gdynia (PL)
(74) Representative: Pawlowska-Bajerska, Justyna

(56) References cited:
- US-A1- 2013 178 913
- US-A1- 2014 148 872
- US-A1- 2020 023 189
- US-A1- 2020 086 047

## Description

The invention refers to the system for improving cognitive functions of the brain, such as memory or attention, using current stimulation and computer devices.

The cortex of the human brain is the outermost structure of the brain that is divided into areas determined in terms of anatomical and functional features. There are areas of the sensory, associative and motor cortex responsible for specific sensory, mental or motor functions. One of the most famous atlases of the human cortex uses numerical designations of areas called Brodmann fields, i.e. the name of the scientist who first marked them. Each field comprises both gyri and sulci in a given area. There are also names of individual fields named after their discoverers, such as Wernicke's field or Broca's field important in speech functions. The higher functions of the human mind, such as declarative memory, are distributed in many cortical and subcortical areas. The cortical areas of the brain crucial for memory and modulation of mental functions remain largely unknown.

It is now possible to map the functions of the cerebral cortex in different areas and to stimulate these areas with electrical current or magnetic field to improve specific functions. Cortical areas showing significant electrophysiological activities during memorization of stimuli can be monitored in real time through special electrode contacts. Brain activity can be measured by invasive techniques such as LFP (Local Field Potential) or ECoG (electrocorticogram) or non-invasive techniques such as EEG (electroencephalogram). The contacts of the electrodes used for the measurement can also be used to stimulate the same areas of the brain by introducing electrical current. Magnetic field stimulation is also possible.

When microelectrodes are used, it is possible to read electrophysiological activity from the surface of the cerebral cortex, its interior and subcortical structures in the form of action potentials sampled at a distance of 50-350 µm from the tip of the electrode, and local electrical fields of ionic currents sampled at a distance of 0.5-3 mm. It is common to use macroelectrodes for intracranial EEG (iEEG) implanted in the cerebral cortex or on its surface, which allows reading of electrophysiological activity with a lower spatial resolution of about 1 cm. The use of an EEG electrode with scalp-mounted contacts allows transcranial recording of collective brain electrophysiological activity with a lower spatial resolution than in the case of implanted electrodes. All of the electrodes listed above can also be used to stimulate the brain by delivering electrical current to selected brain areas.

Methods for spectral analysis of a signal, for example, by applying short-time Fourier transform or a wavelet transformare commonly used to obtain a spectrogram, on the basis of power profiles determined by averaging spectral frequency values in each segment of the spectrogram. A method of filtering the signal with infinite impulse response filters and finite impulse response filters is also commonly applied. There is a well-known method of inductive learning, involving the use of, for example, artificial neural networks. An amplifier is commonly used to amplify electrophysiological signals coming from the brain to values that enable analysis of these signals using digital processing methods, such as, for example, short-time Fourier transform, and to amplify the generated stimulating impulses to values that are safe for cortical surface stimulation, i.e. up to 15 mA and for subcortical stimulation, i.e. 5 mA max. An analog-to-digital converter is commonly used to convert an analog electrophysiological signal into a digital signal. A circular buffer is commonly used to store digital signal samples. Stimulator systems are commonly used to generate a stimulating current with a predetermined amplitude, frequency and pulse duration. A contact selection system with a noise level below 2.5 µV RMS is commonly used to allow the stimulation current to be sent only to selected electrode contacts.

From the patent description EP3247267A2 and the patent description WO2019183046A1, methods of improving cognitive functions, such as human memory, by invasive or non-invasive recording of electrical activity from selected parts of the brain and stimulation in these places are known. These methods are based on electrodes and devices to measure the activity of specific parts of the brain and conduct electrical current to stimulate them. Electrophysiological signals sampled from the brain are transmitted for recording and signal processing to automatically control electrical stimulation to improve memory.

According to the patent description EP3247267, signals can be collected for recording and processing directly from a given part of the brain, while according to the patent description WO2019183046, signals are collected indirectly from other sources of brain activity signals, such as eye pupil dilation. They describe the part of the brain in the lateral temporal cortex that, when electrically stimulated, improves word recall. Stimulation in this part of the brain in an open-circuit mode, i.e. without measuring brain activity, or in a closed-circuit mode, i.e. initiated by measuring and analyzing brain activity while showing word stimuli, increases the average number of words remembered.

From patent descriptions US10363420, US20170113046, US9403010, US20140107728, electrical stimulation is applied in other parts of the brain, within the structures of the medial temporal lobe, such as the hippocampus, entorhinal cortex and their connections with other brain areas.

In the patent description WO09067323, stimulation in the dorsolateral prefrontal cortex (DLPFC) was applied, among others, to improve memory function. Stimulation in these other parts of the brain is used during cognitive activities and memory processes.

From the patent description US2014148872, a device for transcranial stimulation based on two electrodes is known. The disadvantage of this device is the inability to acquire signals of electrophysiological activity, and the lack of possibilities for precise stimulation of brain areas in the anterior ventrolateral prefrontal cortex (anterior VLPFC) between Broca's area, the frontal pole, and the anterior dorsolateral prefrontal cortical Brodmann's areas.

US2020023189 describes a device for intracranial stimulation to improve cognitive abilities. The disadvantage of this device is that it does not detect damaged contacts/wires and does not remove their signals from the analysis process. The device does not allow long-term analysis of stimulation signals and parameters in order to optimize stimulation. This solution does not allow brain stimulation in the area of the anterior ventrolateral prefrontal cortex (anterior VLPFC) between Broca's area (Brodmann areas 44 and 45), the frontal pole (Brodmann areas 10 and 11) and the dorsal anterior lateral prefrontal cortex (Brodmann area 46).

Patent descriptions US20170014630 and US10159839 contain a description of methods of brain stimulation during sleep in order to better consolidate memory traces after a period of cognitive function activity, such as remembering. US 2013/178913 A1 discusses further related systems.

Scientific thorough research has been conducted at the Gdansk University of Technology in the period 01-06-2018 - 31-05-2021, covering the neurophysiological mechanisms of thought processes and memory through the use of innovative technologies to measure and stimulate human brain activity. The results of these studies show that there are significantly greater differences in activity in the theta frequency band of the brain waves (2-8 Hz) between successful and unsuccessful human memory trials. The differences found in memory performance were greater than in other parts of the brain (including the lateral temporal cortex) or in higher frequency bands of the brainwave spectrum. Based on the analysis the invention was provided.

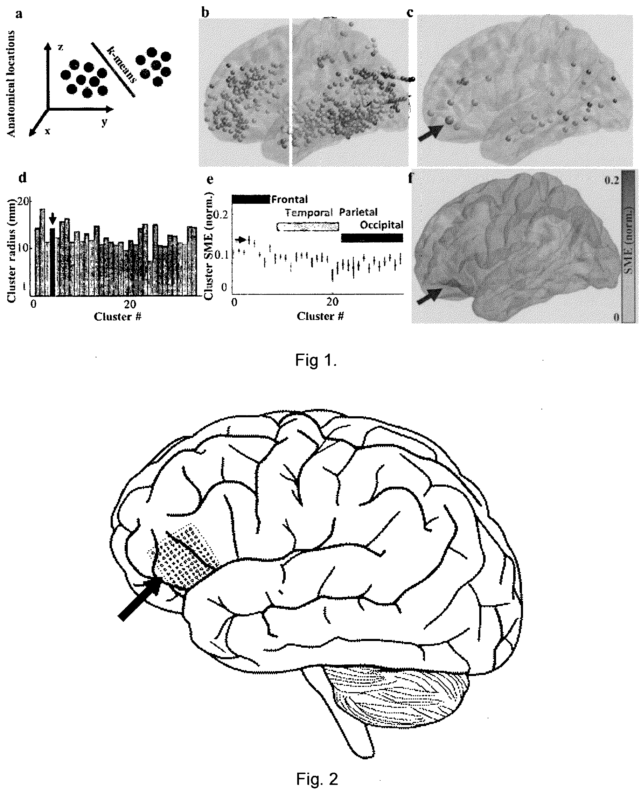

This is illustrated in Fig. 1, where Fig. 1A shows that all active electrodes implanted in this study were automatically grouped based on their anatomical location coordinates based on the k-means method, Fig. 1B shows that the automatically grouped electrode clusters show a greater density and magnitude of difference in the signal of memory (ball size) in the prefrontal cortex, Fig. 1C shows that the centroids of each cluster identify one cluster with the highest mean difference in recall in the anterior prefrontal cortex (arrow). Fig. 1D shows that the anatomical distribution of each cluster reveals a relatively small size of the discovered area (arrow) with a radius of 4 mm. Fig. 1E shows that a comparison of averaged differences in memory effects between anatomically aligned clusters confirms significantly higher values for the discovered prefrontal clusters than most other clusters, and that adjacent prefrontal clusters also show relatively high mean differences compared to progressively more anatomically distant clusters. Figure 1F shows a universal brain surface, which summarizes the interpolated difference values, which in turn confirm the same anatomically distinct cluster (arrow) between the frontal pole, Broca's area, and the lateral prefrontal cortex. This localized area of the brain is anatomically accessible to invasive and non-invasive techniques for sensing activity and for brain stimulation.

The invention is as defined by the claims.

The use of the system to improve the cognitive functions of the human brain allows you to improve cognitive functions, i.e. support the memorization process, by electrical stimulation of the brain in the area of the prefrontal cortex: anterior ventro-lateral prefrontal cortex; anterior VLPFC between Broca's area ( Brodmann area 44 and 45), frontal pole (Brodmann area 10 and 11) and anterior dorsolateral prefrontal cortex (Brodmann area 46).

The invention is explained in detail in the examples of implementation and in the drawing, in which Fig. 1 shows schematically the results of scientific research carried out at the Gdańsk University of Technology - source:
https://pubmed.ncbi.nlm.nih.gov/35785617/, and Fig. 2 shows the arrangement of electrode contacts. Fig. 3 shows a general scheme of the invention for improving cognitive functions, and Fig. 4, Fig. 5, Fig. 6, Fig. 7 show embodiments of the systems..

### Example 1

### System construction

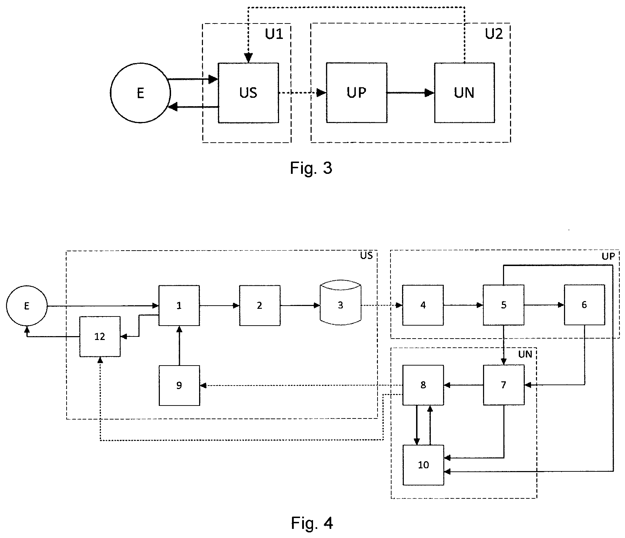

As shown in Figures 3 and 4, the cognitive enhancement system comprises of a known stimulating electrode with 100 contacts conducting electrical current (E), which are geometrically spaced equidistantly in a grid with a total area of maximum 5 cm x 5 cm. The grid is centered in the middle of an activity area in the anterior ventrolateral prefrontal cortex, which is indicated in Fig. 1F, carefully delineated beforehand using an invasive or non-invasive technique for measuring EEG signals. The arrangement of the contacts is shown in Fig. 2. The contacts are placed on a biocompatible and non-reactive material using, for example, polyamide lithography techniques. The diameter of the contacts is small enough, less than 100 micrometers, to receive LFP signals from individual cortical columns, and large enough to allow safe stimulation currents, i.e. a maximum of 15 mA for cortical stimulation and 5 mA for deep brain stimulation.

As shown in Fig. 3, the electrode contacts E are connected to the US acquisition and stimulation system, which is connected wirelessly via Bluetooth to the UP processing system on a tablet or smartphone, which in turn is connected to the UN analysis system implemented in the same form, which is wirelessly connected to the US acquisition and stimulation system. The US acquisition and stimulation system in this example is one stimulator device U1, and the UP and UN systems are part of the second computing device U2. The US, UP and UN systems are detailed below.

As shown in Fig. 4, the US acquisition and stimulation system comprisi: a contact selection system 12 with a noise level below 2.5 µVRMS, an amplifier 1 that amplifies the signal obtained from the electrode contacts and the signal generated by the generator 9 to safe values in the case of cortical stimulation, i.e. max. 15 mA and max. 5 mA in the case of subcortical stimulation, analog-to-digital converter 2, buffer 3 that is implemented in the form of a circular buffer with a length of at least 800 samples and being part of the operational memory of the system, and generator system 9 that is used to generate a stimulating current with a given amplitude, frequency and pulse duration. The stimulating electrode E is wired to the amplifier circuit 1, which is connected to the analog-to-digital converter 2, which is connected to the buffer 3, which in turn is connected wirelessly via the Bluetooth protocol to the filtration circuit 4 located in the UP processing circuit. The filtration system 4, which divides the signal into spectral frequency bands using filters with an infinite impulse response for theta, alpha and beta bands and filters with a finite impulse response for low and high gamma bands and higher frequency bands, is connected to the spectral power measurement system 5, wherein for signals at least in the theta frequency band, a spectrogram is determined by a spectral frequency analysis method, and then a spectral power profile is determined by averaging the spectral frequency values in each segment of the spectrogram. The spectral power measurement system 5 is connected to the fault detection system 6, in which defective contacts of the stimulating electrode E or wires are detected, using a spectral frequency analysis method of signal noise detection with a signal coming from the electrical network, comprisins, for example, in comparison with the threshold value of the median difference for each segment spectrogram and the median of these medians and using the spectral frequency method of detecting epileptic / non-electrophysiological activity resulting in the Gibbs effect, comprises, for example, in calculating the quotient of the mean value from the sum of values in the spectrogram and the median from the sum of values in the spectrogram, and then comparing the result with the threshold value of 2 for a very subtle Gibbs effect and a value of 18 for a strong Gibbs effect. The spectral power measurement system 5 is also connected to the threshold system 7, located in the UN analysis system, in which the threshold system 7 for undamaged electrode contacts is determined by simple rules - conditional instructions, in which spectral frequency bands, at least for theta band and for which electrode contacts E the power thresholds were exceeded. The spectral power measurement system 5 is also connected to the optimization system 10, which performs long-term analysis of such data as the signal spectral power profiles at least in theta bands and the identifiers of the stimulating electrode contacts E, where the spectral power thresholds were exceeded, obtained from the spectral power measurement system 5, in to determine the optimal set of pulse amplitude, frequency and duration values, and stimulation locations, based on machine learning, e.g. using an inductive learning method. The threshold system 7 is connected with the decision system 8 and the optimization system 10. The decision system 8 is based on a set of simple rules - conditional instructions makes a decision on which contacts of the stimulating electrode E the stimulating signal should be sent and what parameters it should have, i.e. the amplitude, duration of the electrical impulse and its frequency. The decision circuit 8 is wirelessly connected to the generator circuit 9, in which an electric impulse of a predetermined amplitude, duration and frequency is generated, the contact selection circuit 12 and the optimization circuit 10. The generator circuit 9 is connected to the amplifier 1.

### Methodology - methods performed on the invention - the whole system

Signal from stimulator electrode E, spaced geometrically equidistant from each other in a 10x10 grid, over a total area of maximum 5 cm x 5 cm centered in the area of activity of the anterior ventrolateral prefrontal cortex, shown in Fig. 1F, carefully traced beforehand with invasive or non-invasive technique of measuring EEG signals, is sent to the amplifier 1, which together with the contact selection system 12, generator 9, analog-to-digital converter 2 and buffer 3, implemented in the form of a circular buffer with a length of at least 800 samples and being part of the operational memory system, are part of the US acquisition and stimulation system implanted in the head or chest under the collarbone, as in the case of Deep Brain Stimulation (DBS) therapy devices. The US system is powered by a wirelessly charged battery or a long-term surgical replacement battery, as in the case of DBS therapy devices. Signals amplified in the amplifier 1, to the value of amplitudes enabling analysis using processing methods such as short-time Fourier transform, are sent to the analog-to-digital converter 2, where they are converted into digital form, and then N signal samples are sent to buffer 3 from which they are obtained and sent wirelessly in Bluetooth technology to the filtering system 4, in which the sampled signals are divided into spectral frequency bands using filters with an infinite impulse response for theta, alpha and beta bands and filters with a finite impulse response for low and high spectral frequency gamma bands and higher frequency bands. The signals divided into spectral frequency bands are sent to the spectral power measurement system 5, in which, by averaging the spectral frequency values in each segment of the spectrogram obtained using the signal spectral frequency analysis method, the signal spectral power profile is determined in each of the spectral frequency bands, at least in the theta band. The determined spectrograms in each of the spectral frequency bands are sent to the fault detection system 6, in which faulty contacts or wires are detected, using the method of detecting electrical noise in the signal based on the analysis of the spectrogram by comparison with the threshold value of the difference between the median for each segment of the spectrogram and the median of these medians and using the method of detecting epileptic / non-electrophysiological activity. Power profiles determined in the spectral power measurement system 5 and information about damaged contacts/wires determined in the fault detection system 6 are sent to the threshold circuit 7, in which, for undamaged contacts of the stimulating electrode, E is determined using simple rules in which spectral frequency bands and for which of the pacing lead E contacts, spectral power thresholds have been exceeded. When using the invention to improve cognition processes, threshold values are established individually during patient memory testing in clinical conditions after the operation and entered by the doctor in the application running on the U2 computer device and blocked with a password so that the user cannot change them on their own. For example, a theta band spectral power above the threshold value with a simultaneous gamma band power value below the threshold value in the area of the anterior ventrolateral prefrontal cortex (VLPFC) between Broca's area - Brodmann area 44 and 45, the pole frontal - Brodmann's area 10 and 11 and anterior dorsolateral prefrontal cortex - Brodmann's area 46 observed by the doctor during memory tests in the patient, in the interval of 0-500 ms from the display of the word on the screen, indicate an attempt to remember. The occurrence of the spectral power relation described above in everyday life on the given contacts of the stimulating electrode E means that current stimulation can be performed on these contacts to enhance the cognitive process. Information about the relations between the spectral power values in each spectral frequency band and the threshold values is transferred to the decision system 8, operating on the basis of a set of simple rules - conditional instructions, and to the optimization system 10, operating on the basis of machine learning, e.g. using the inductive learning method. Based on the information about the contacts of the stimulating electrode E, on which the thresholds were exceeded, and based on the spectral power values and data on the optimal parameters of the stimulating signal and stimulation sites from the optimization system 10, the decision-making system 8 sends wirelessly via Bluetooth technology to the generator system 9 information about the parameters of the stimulating signal, such as the frequency and amplitude of the current and the duration of the pulse to be generated by the generator 9. The decision circuit 8 also sends wirelessly via Bluetooth technology to the contact selection circuit 12 information on which contacts of the stimulating electrode E the stimulating signal should be sent generated by the generator 9 and amplified in the amplifier 1. The decision circuit 8 also stores in the memory of the optimization circuit 10 the current parameters of the stimulating signal and information on which contacts of the stimulating electrode E the stimulating signal should be sent. The memory of the optimization circuit 10 also stores information from the threshold circuit 7 about which spectral frequency bands and for which contacts of the stimulating electrode E the spectral power thresholds were exceeded. The memory of the optimization system 10 also stores the signal strength profiles in each of the spectral frequency bands obtained from the spectral power measurement system 5. Additionally, the optimization system 10 stores optional feedback from the user about whether the applied stimulation did not cause unpleasant sensations and whether it actually contributed to improve memory. Feedback from the user is provided via an application running on U2's computer device, i.e. a tablet. The optimization system 10 performs a long-term analysis of this data to determine the optimal set of parameters and stimulation sites, based on machine learning, e.g. using an inductive learning method.

### Example 2

### System construction

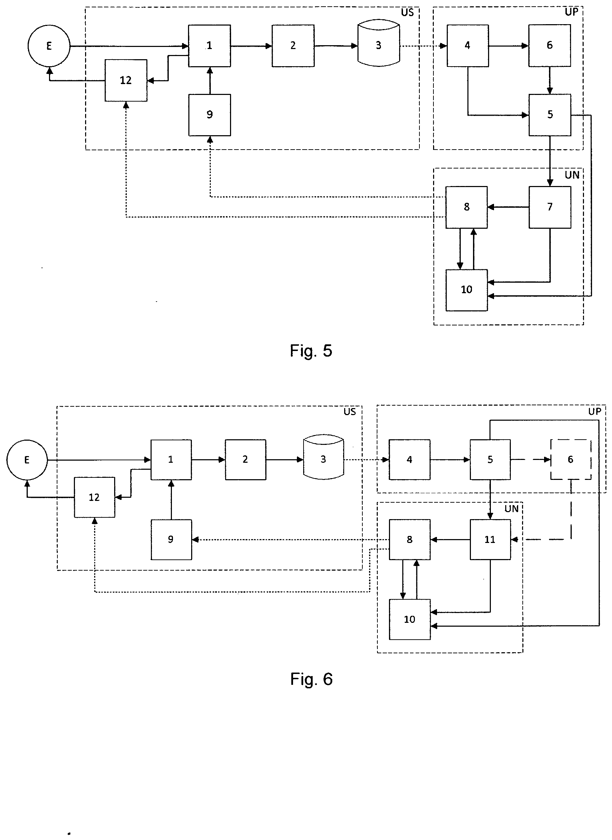

As shown in Figures 3 and 5, the cognitive enhancement system comprises a known stimulating electrode with 100 current-carrying contacts E, geometrically spaced at equal distances from each other in a 10x10 grid, over a total area of maximum 5 cm x 5 cm centered in the center of the area of activity of the anterior ventrolateral prefrontal cortex indicated in Fig. 1F, carefully delineated in advance by an invasive or non-invasive technique for measuring EEG signals. The arrangement of the contacts is shown in Fig. 2. The contacts are placed on a biocompatible and non-reactive material using, for example, polyamide lithography techniques. The diameter of the contacts is small enough, less than 100 micrometers, to receive LFP signals from individual cortical columns, and large enough to allow safe stimulation currents, i.e. a maximum of 15 mA for cortical stimulation and 5 mA for deep stimulation .

As shown in Fig. 3, the contacts of the stimulating electrode E are connected to the US acquisition and stimulation system, which is connected wirelessly via Bluetooth to the UP processing system on a tablet or smartphone, which in turn is connected to the UN analysis system implemented in the same form, which is wirelessly connected to the US acquisition and stimulation system. The US acquisition and stimulation system in this example is one stimulator device U1, and the UP and UN systems are part of the second computing device U2. The US, UP and UN systems are detailed below.

As shown in Fig. 5, the US acquisition and stimulation system comprisis a contact selection system 12 with a noise level below 2.5 µVRMS, an amplifier 1 that amplifies the signal obtained from the electrode contacts and the signal generated by the generator 9 to safe values in the case of cortical stimulation. , i.e. max. 15 mA and in the case of subcortical stimulation, i.e. max. 5 mA, analog-to-digital converter 2, buffer 3, implemented in the form of a circular buffer with a length of at least 800 samples and being part of the operational memory of the system, and generator system 9, used to generate a stimulating current with a given amplitude, frequency and pulse duration. The stimulating electrode E is wired to the amplifier circuit 1, which is connected to the analog-to-digital converter 2, which is connected to the buffer 3, which in turn is connected wirelessly to the filtration circuit 4 located in the UP processing circuit. Filtering system 4, which divides the signal into spectral frequency bands using infinite impulse response filters for theta, alpha and beta bands and finite impulse response filters for low and high gamma wave bands and higher frequency bands, is connected to
a spectral power measurement system 5, in which for signals at least in the theta frequency band, a spectrogram is determined by means of a spectral frequency analysis method, and then a spectral power profile is determined by averaging the spectral frequency values in each segment of the spectrogram. The filtering system 4 is also connected to the fault detection system 6. The fault detection system 6, which in this example implements a time method comprise e.g. difference with the threshold value, is connected to the spectral power and/or field potential measurement circuit 5, which in turn is connected to the threshold circuit 7 and the optimization circuit 10, operating as in example 1. Further, the circuit connections are the same as in example 1 .

### Methods performed on the invention - the whole system

Signal from stimulator electrode E, spaced geometrically equidistant from each other in a 10x10 grid, over a total area of maximum 5 cm x 5 cm centered in the area of activity of the anterior ventrolateral prefrontal cortex, shown in Fig. 1F, carefully traced beforehand with invasive or non-invasive EEG signal measurement technique, is sent to the amplifier 1, which together with the contact selection system 12, generator 9 and buffer 3, implemented in the form of a circular buffer with a length of at least 800 samples and being part of the operational memory of the system, are part of the acquisition and UA stimulation implanted in the head or chest under the collarbone, such as with Deep Brain Stimulation (DBS) therapy devices. The UA chip is powered by a wirelessly rechargeable battery or a long-term surgically replaceable battery such as DBS therapy devices. The signals amplified in the amplifier 1, to the amplitude values enabling signal analysis using processing methods such as short-time Fourier transform, are sent to the analog-to-digital converter 2, which converts the signal into digital form, and then to the buffer 3, from which the signals are obtained in a sampled form and sent wirelessly in Bluetooth technology to the filtration system 4, in which they are divided into spectral frequency bands using infinite impulse response filters for theta, alpha and beta bands and finite impulse response filters for low and high gamma wave bands and higher frequency bands. The signals, divided into spectral frequency bands, are sent to the spectral power measurement system 5, in which, by averaging the spectral frequency values in each segment of the spectrogram obtained using the spectral frequency analysis method, the signal spectral power profile is determined in each of the spectral frequency bands, at least in the theta band. Signals divided according to spectral frequency bands in the filtration system 4 are also sent to the fault detection system 6, in which faulty contacts or wires are detected using the time method of signal noise detection, e.g. by subtracting the signal energy in the selected band from the signal energy in the adjacent higher frequency band, containing the fundamental spectral frequency or current harmonic, and comparing the obtained difference with the threshold. In the spectral power and/or field potential measurement system 5, the signal spectral power profile in each of the spectral frequency bands is determined by averaging the spectral frequency values in each segment of the spectrogram obtained using the spectral frequency analysis method and/or the number of potentials is determined by determining the average amplitude in the time domain within a segment of n samples, adding a threshold value to it and identifying the places where the amplitude exceeds this sum. The determined spectral power profiles in each of the spectral frequency bands and/or the number of potentials are sent to the threshold system 7, in which, using simple rules, it is determined in which spectral frequency bands and for which contacts of the stimulating electrode E the spectral power thresholds and/or the number of potentials were exceeded. As in example 1, the determined spectral power profiles and/or field potentials from the spectral power and/or field potential measurement system 5 are also sent to the optimization system 10, operating on the basis of machine learning, e.g. using the inductive learning method, after then proceeding as described in Example 1.

### Example 3

### System construction

As shown in Figures 3 and 6, the cognitive enhancement system comprises a stimulating electrode with 100 E-conducting contacts geometrically spaced at equal distances from each other in a 10 x 10 grid, with a total area of maximum 5 cm x 5 cm centered the area of activity of the anterior ventrolateral prefrontal cortex indicated in Fig. 1F, carefully delineated in advance by an invasive or non-invasive technique for measuring EEG signals. The arrangement of the contacts is shown in Fig. 2. The contacts are placed on a biocompatible and non-reactive material using, for example, polyamide lithography techniques. The diameter of the contacts is small enough, less than 100 micrometers, to receive LFP signals from individual cortical columns, and large enough to allow safe stimulation currents, i.e. a maximum of 15 mA for cortical stimulation and 5 mA for deep stimulation .

As shown in Fig. 3, the contacts of the stimulating electrode E are connected to the US acquisition and stimulation system, which is connected wirelessly via Bluetooth to the UP processing system on a tablet or smartphone, which in turn is connected to the UN analysis system implemented in the same form, which is wirelessly connected to the US acquisition and stimulation system. The US acquisition and stimulation system in this example is one stimulator device U1, and the UP and UN systems are part of the second computing device U2. The US, UP and UN systems are detailed below.

As shown in Fig. 6, the US acquisition and stimulation system comprises a contact selection system 12 with a noise level below 2.5 pVRMS, an amplifier 1 that amplifies the signal obtained from the electrode contacts and the signal generated by the generator 9 to safe values in the case of cortical stimulation. , i.e. max. 15 mA and in the case of subcortical stimulation, i.e. max. 5 mA, analog-to-digital converter 2, buffer 3, implemented in the form of a circular buffer with a length of at least 800 samples and being part of the operational memory of the system, and generator system 9, used to generate a stimulating current with a given amplitude, frequency and pulse duration. The stimulating electrode E is wired to the amplifier 1, which is connected to the analog-to-digital converter 2, which is connected to the buffer 3, which in turn is connected wirelessly to the filtration system 4 located in the UP processing system. The filtration system 4, which divides the signal into spectral frequency bands using filters with an infinite impulse response for theta, alpha and beta bands and filters with a finite impulse response for low and high gamma bands and higher frequency bands, is connected to the spectral power measurement system 5, wherein for signals at least in the theta frequency band, a spectrogram is determined by a spectral frequency analysis method, and then a spectral power profile is determined by averaging the spectral frequency values in each segment of the spectrogram. The spectral power measurement system 5 is connected to the classification system 11, the optimization system 10 and optionally to the fault detection system 6. The optional fault detection system 6 is connected to the classification system 11 and the optimization system 10. The classification system 11 is connected to the decision system 8 and the optimization system 10. Next, the circuit connections are the same as in example 1.

### Methods performed on the invention - the whole system

Signal from stimulator electrode E, spaced geometrically equidistant from each other in a 10x10 grid, over a total area of maximum 5 cm x 5 cm centered in the area of activity of the anterior ventrolateral prefrontal cortex, shown in Fig. 1F, carefully traced beforehand with invasive or non-invasive EEG signal measurement technique, is sent to the amplifier 1, which together with the contact selection system 12, the analog-to-digital converter 2, the generator 9 and the buffer 3, implemented in the form of a circular buffer with a length of at least 800 samples and being part of the operational memory system, are part of the US acquisition and stimulation system implanted in the head or chest under the collarbone, as in the case of Deep Brain Stimulation (DBS) therapy devices. The US system is powered by a wirelessly charged battery or a long-term surgical replacement battery, as in the case of DBS therapy devices. The signals amplified in the amplifier 1, to the amplitude values enabling signal analysis using processing methods such as short-time Fourier transform, are sent to the analog-to-digital converter 2, where they are converted into digital form and sent to buffer 3. lengths of N samples obtained from buffer 3 are sent wirelessly in Bluetooth technology to the filtering system 4, in which they are divided into spectral frequency bands using filters with an infinite impulse response for theta, alpha and beta bands and filters with a finite impulse response for low and high bands gamma waves and higher frequency bands. The signals, divided into spectral frequency bands, are sent to the spectral power measurement system 5, in which, by averaging the spectral frequency values in each segment of the spectrogram obtained using the spectral frequency analysis method, the signal spectral power profile is determined in each of the spectral frequency bands, at least in the theta band. Spectral power profiles in each of the spectral frequency bands determined in the power measurement system 5, by averaging the spectral frequency values in each segment of the spectrogram obtained using the spectral frequency analysis method, and/or potentials determined in the time domain by calculating the average amplitude within the segment of n samples, adding to it threshold value and identification of places where the amplitude exceeds this sum are sent to the classification system 11, operating on the basis of artificial neural networks, which classifies into classes, each of which determines a set of parameters of the stimulating signal, such as the amplitude and frequency of the current and the duration of the pulse, and contacts to be stimulated. Spectrograms from the spectral power measurement system 5 are optionally sent to the fault detection system 6, in which faulty contacts or wires are detected using the spectral frequency method of noise detection, e.g. methods for detecting epileptic / non-electrophysiological activity resulting in the Gibbs effect, consisting, for example, in calculating the quotient of the mean value from the sum of the values in the spectrogram and the median from the sum of the values in the spectrogram, and then comparing the result with the threshold value of 2 for a very subtle Gibbs effect and the value of 18 for a strong one the Gibbs effect. Information about faulty contacts or wires is sent to the classifier 11. The optionality of the system 6 comprises in the fact that the classifier 11 can also classify the signal received from the spectral power measurement system 5 in terms of damaged contacts or wires. Then, the signals are sent bypassing the fault detection system 6. Based on the class identified in the classification system 11 on the basis of information sent from the spectral power measurement system 5 and data on the optimal parameters of the stimulating signal and stimulation locations from the optimization system 10, operating based on machine learning e.g. using the inductive learning method, the decision-making system 8 sends wirelessly via Bluetooth technology to the generator system 9 information about the parameters of the stimulating signal to be generated by the generator 9. The memory of the optimization system 10 also stores information from the classification system 11 about the selected class defining a set of parameters of the stimulating signal and contacts on which stimulation should be performed. The memory of the optimization system 10 also stores the signal spectral power profiles in each of the spectral frequency bands, obtained from the spectral power measurement system 5.

### Example 4

### System construction

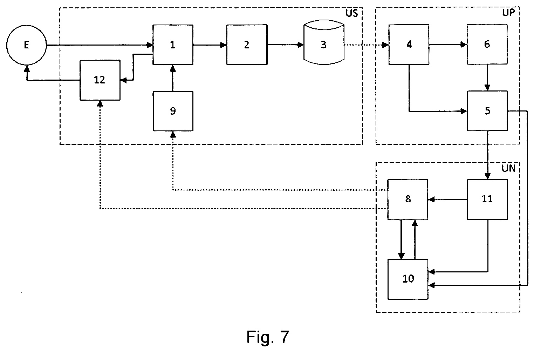

As shown in Figures 3 and 7, the cognitive enhancement system comprises a stimulating electrode with 100 E-conducting contacts geometrically spaced at equal distances from each other in a 10 x 10 grid, over a total area of maximum 5 cm x 5 cm centered in the area the activity of the anterior ventrolateral prefrontal cortex, indicated in Fig. 1F, carefully traced beforehand using an invasive or non-invasive technique for measuring EEG signals. The arrangement of the contacts is shown in Fig. 2. The contacts are placed on a biocompatible and non-reactive material using, for example, polyamide lithography techniques. The diameter of the contacts is small enough, less than 100 micrometers, to receive LFP signals from individual cortical columns, and large enough to allow safe stimulation currents, i.e. a maximum of 15 mA for cortical stimulation and 5 mA for deep stimulation .

As shown in Fig. 3, the contacts of the stimulating electrode E are connected to the US acquisition and stimulation system, which is connected wirelessly via Bluetooth to the UP processing system on a tablet or smartphone, which in turn is connected to the UN analysis system implemented in the same form, which is wirelessly connected to the US acquisition and stimulation system. The US acquisition and stimulation system in this example is one stimulator device U1, and the UP and UN systems are part of the second computing device U2. The US, UP and UN systems are detailed below.

As shown in Fig. 7, the US acquisition and stimulation system comprises a contact selection system 12 with a noise level below 2.5 µVRMS, an amplifier 1 that amplifies the signal obtained from the electrode contacts and the signal generated by the generator 9 to safe values in the case of cortical stimulation. , i.e. max. 15 mA and in the case of subcortical stimulation, i.e. max. 5 mA, analog-to-digital converter 2, buffer 3, implemented in the form of a circular buffer with a length of at least 800 samples and being part of the operational memory of the system, and generator system 9, used to generate a stimulating current with a given amplitude, frequency and pulse duration. The stimulating electrode E is connected to the amplifier 1, which is connected to the analog-to-digital converter 2, which in turn is connected to the buffer 3, which is connected wirelessly to the filtration system 4 located in the UP processing system. The filtration system 4, which divides the signal into spectral frequency bands using filters with an infinite impulse response for theta, alpha and beta bands and filters with a finite impulse response for low and high gamma bands and higher frequency bands, is connected to the spectral power measurement system 5, wherein for signals at least in the theta frequency band, a spectrogram is determined by a spectral frequency analysis method, and then a spectral power profile is determined by averaging the spectral frequency values in each segment of the spectrogram. The filtering system 4 is also connected to the fault detection system 6. The fault detection system 6, which in this example implements a time method consisting in e.g. the difference with the threshold value, is connected to the spectral power and/or field potential measurement circuit 5, which in turn is connected to the classifier 11 and the optimization circuit 10. The classifier 11 is connected to the decision circuit 8 and the optimization circuit 10. are the same as in example 2.

### Methods performed on the invention - the whole system

Signal from stimulator electrode E with contacts geometrically spaced equidistant from each other in a 10x10 grid, over a total area of maximum 5 cm x 5 cm with the center at the center of the area of activity of the anterior ventrolateral prefrontal cortex, shown in Fig. 1F, carefully traced beforehand with using an invasive or non-invasive technique for measuring EEG signals, is sent to the amplifier 1, which together with the contact selector 12, the analog-to-digital converter 2, the generator 9 and the buffer 3, implemented in the form of a circular buffer with a length of at least 800 samples and being part of memory of the system, are part of the US acquisition and stimulation system implanted in the head or chest under the collarbone, as in the case of Deep Brain Stimulation (DBS) therapy devices known from the state of the art. The US system is powered by a wirelessly charged battery or a long-term surgical replacement battery, as in the case of DBS therapy devices. The signals converted into digital form in the analog-to-digital converter 2 are sent to the buffer 3, from which they are acquired and sent wirelessly in Bluetooth technology to the filtration system 4, where they are divided into spectral frequency bands using infinite impulse response filters for theta bands , alpha and beta, and finite impulse response filters for low and high gamma bands and higher frequency bands. The signals, divided into spectral frequency bands, are sent to the spectral power measurement system 5, in which, by averaging the spectral frequency values in each segment of the spectrogram obtained using the spectral frequency analysis method, the signal spectral power profile is determined in each of the spectral frequency bands, at least in the theta band. Signals divided according to spectral frequency bands in the filtration system 4 are also sent to the fault detection system 6, in which faulty contacts or wires are detected using the time method of signal noise detection, e.g. by subtracting the signal energy in the selected band from the signal energy in the adjacent higher frequency band, containing the fundamental spectral frequency or current harmonic, and comparing the obtained difference with the threshold. In the spectral power and/or field potential measurement system 5, the signal spectral power profile in each of the spectral frequency bands is determined by averaging the spectral frequency values in each segment of the spectrogram obtained using the spectral frequency analysis method and/or the field potentials are determined by determining the average amplitude in the time domain within a segment of n samples, adding a threshold value to it and identifying the places where the amplitude exceeds this sum. The determined spectral power profiles in each of the spectral frequency bands and/or potentials are sent to the classification system 11, operating on the basis of artificial neural networks, which classifies into classes, each of which determines a set of parameters of the stimulating signal, such as the amplitude and frequency of the current and the time pulse duration, and the contacts to be stimulated. Based on the class identified in the classification system 11 on the basis of information sent from the spectral power and/or field potential measurement system 5 and data on the optimal parameters of the stimulating signal and stimulation locations from the optimization system 10, operating based on machine learning, e.g. Inductive learning, the decision-making system 8 transmits, wirelessly, using Bluetooth technology, to the generator system 9 information about the parameters of the stimulating signal to be generated by the generator 9. The memory of the optimization system 10 also stores information from the classification system 11 about the selected class defining the set of parameters of the stimulating signal and contacts to be stimulated. The memory of the optimization system 10 also stores the signal spectral power profiles and/or the number of potentials in each of the spectral frequency bands obtained from the spectral power and/or field potential measurement system 5. The procedure is the same as in example 2.

## Claims

1. System for improving cognitive functions of the human brain in the area of the anterior ventro-lateral prefrontal cortex (anterior VLPFC) between Broca's area (Brodmann area 44 and 45), frontal pole (Brodmann area 10 and 11) and anterior dorsolateral prefrontal cortex (Brodmann area 46) and for stimulation in these areas of the brain comprising at least one stimulating electrode (E), which has at least one contact capable of acquiring an LFP signal or which is a single contact EEG electrode adapted to be non-invasively mounted on scalp or which is an iEEG macroelectrode with one contact adapted to be mounted in the cerebral cortex or on its surface, **characterized in that** the system comprises an acquisition and stimulation system (US), a processing system (UP), an analysis system (UN), wherein the stimulating electrode (E) is connected by wire or wirelessly and also bi-directionally to the acquisition and stimulation system (US), which is connected to the processing system (UP), which is connected to the analysis system (UN), which is connected back to the acquisition and stimulation system (US), and additionally . wherein the acquisition and stimulation system (US) comprises an amplifier (1), an analog-to-digital converter (2), a buffer (3), a generator (9) and a contact selection circuit (12), while the amplifier (1) is connected to the analog-to-digital converter (2), which is connected to the buffer (3), and the processing system (UP) comprises a filtration system (4), a spectral power and/or field potential measurement system (5) and a fault detection system (6), while the buffer (3) is connected with the filtration system (4) and the filtration system (4) is connected to the fault detection system (6) through the spectral power and/or field potential measurement system (5)**,** in which a method of spectral frequency analysis of the signal is implemented, whereby the spectral power and/or field potential measurement system (5) is connected to a threshold system (7) located in the analysis system (UN), and moreover the threshold system (7) is connected to a decision-making system (8) being part of the analysis system (UN), and the spectral power and/or field potential measurement system (5) and the threshold system (7) are connected to an optimization system (10) being part of the analysis system (UN) which is bi-directionally connected to the decision-making system (8), which is connected to an actuator (9) in the acquisition and stimulation system (US), wherein the actuator (9) is connected to the amplifier (1), and the decision circuit (8) is connected to the contact selection circuit (12), the amplifier (1) is also connected to the contact selection circuit (12), and the contact selection circuit (12) is connected to the stimulating electrode (E).

2. The system according to claim 1, wherein the filtration system (4) is connected to the spectral power and/or field potential measurement system (5).

3. The system according to claim 1 wherein the spectral power and/or field potential measurement system (5) is connected to a classification system (11) located in the analysis system (UN), while the classification system (11) is connected to the decision-making system (8), and moreover, the spectral power and/or field potential measurement system (5) and the classification system (11) are connected to the optimization system (10).

4. The system according to claim 3, wherein the spectral power and/or field potential measurement system (5) is connected via the fault detection system (6) to the classification system (11), while the power and/or field potential measurement system (5) is also directly connected to classification system (11).

5. The system according to claim 1 wherein the spectral power and/or field potential measurement system (5) is connected through a classification system (11) to the decision system (8).

## Patentansprüche

1. System zur Verbesserung der kognitiven Funktionen des menschlichen Gehirns im Bereich des anterioren ventrolateralen präfrontalen Kortex (anteriorer VLPFC) zwischen dem Broca-Areal (Brodmann-Areale 44 und 45), dem frontalen Pol (Brodmann-Areale 10 und 11) und dem anterioren dorsolateralen präfrontalen Kortex (Brodmann-Areal 46) und zur Stimulation in diesen Bereichen des Gehirns, umfassend mindestens eine Stimulationselektrode (E), die mindestens einen Kontakt aufweist, der ein LFP-Signal erfassen kann, oder eine Einzelkontakt-EEG-Elektrode ist, die zur nichtinvasiven Anbringung an der Kopfhaut geeignet ist, oder eine iEEG-Makroelektrode mit einem Kontakt ist, der zur Anbringung in der Großhirnrinde oder auf deren Oberfläche geeignet ist, **dadurch gekennzeichnet, dass** das System ein Erfassungs- und Stimulationssystem (US), ein Verarbeitungssystem (UP) und ein Analysesystem (UN) umfasst, wobei die Stimulationselektrode (E) über Kabel oder drahtlos und auch bidirektional mit dem Erfassungs- und Stimulationssystem (US) verbunden ist, das mit dem Verarbeitungssystem (UP) verbunden ist, das mit dem Analysesystem (UN) verbunden ist, das wiederum mit dem Erfassungs- und Stimulationssystem (US) verbunden ist, und wobei zusätzlich das Erfassungs- und Stimulationssystem (US) einen Verstärker (1), einen Analog-Digital-Wandler (2), einen Puffer (3), einen Generator (9) und eine Kontaktwahlschaltung (12) umfasst, wobei der Verstärker (1) mit dem Analog-Digital-Wandler (2) verbunden ist, der mit dem Puffer (3) verbunden ist, und das Verarbeitungssystem (UP) ein Filtersystem (4), ein Spektralleistungs- und/oder Feldpotenzial-Messsystem (5) und ein Fehlererkennungssystem (6) umfasst, wobei der Puffer (3) mit dem Filtersystem (4) verbunden ist und das Filtersystem (4) über das Spektralleistungs- und/oder Feldpotenzial-Messsystem (5) verbunden ist, in dem ein Verfahren zur spektralen Frequenzanalyse des Signals implementiert ist, wobei das Spektralleistungs- und/oder Feldpotenzial-Messsystem (5) mit einem im Analysesystem (UN) befindlichen Schwellwertsystem (7) verbunden ist und das Schwellwertsystem (7) außerdem mit einem zum Analysesystem (UN) gehörenden Entscheidungssystem (8) verbunden ist, und das Spektralleistungs- und/oder Feldpotenzial-Messsystem (5) und das Schwellwertsystem (7) mit einem Optimierungssystem (10) verbunden sind, das Teil des Analysesystems (UN) ist, welches bidirektional mit dem Entscheidungssystem (8) verbunden ist, das mit einem Aktuator (9) im Erfassungs- und Stimulationssystem (US) verbunden ist, wobei der Aktuator (9) mit dem Verstärker (1) verbunden ist und die Entscheidungsschaltung (8) mit der Kontaktwahlschaltung (12) verbunden ist und die Kontaktwahlschaltung (12) mit der Stimulationselektrode (E) verbunden ist.

2. System nach Ansprü che 1, wobei das Filtersystem (4) mit dem Spektralleistungs- und/oder Feldpotenzial-Messsystem (5) verbunden ist.

3. System nach Ansprü ch 1, **dadurch gekennzeichnet** das Spektralleistungs- und/oder Feldpotenzial-Messsystem (5) mit einem Klassifizierungssystem (11) verbunden ist, das sich im Analysesystem (UN) befindet, während das Klassifizierungssystem (11) mit dem Entscheidungssystem (8) verbunden ist, und wobei darüber hinaus das Spektralleistungs- und/oder Feldpotenzial-Messsystem (5) und das Klassifizierungssystem (11) mit dem Optimierungssystem (10) verbunden sind.

4. System nach Ansprüch 3, **dadurch gekennzeichnet** das Spektralleistungs- und/oder Feldpotenzial-Messsystem (5) mit dem Fehlererkennungssystem (6) und dem Klassifizierungssystem (11) verbunden ist, während das Leistungs- und/oder Feldpotenzial-Messsystem (5) auch direkt mit dem Klassifizierungssystem (11) verbunden ist.

5. System nach Ansprüch 1, **dadurch gekennzeichnet** das Spektralleistungs- und/oder Feldpotenzial-Messsystem (5) über ein Klassifizierungssystem (11) mit dem Entscheidungssystem (8) verbunden ist.

## Revendications

1. Système destiné à améliorer les fonctions cognitives du cerveau humain dans la zone du cortex préfrontal ventro-latéral antérieur (VLPFC antérieur) entre l'aire de Broca (aires de Brodmann 44 et 45), le pôle frontal (aires de Brodmann 10 et 11) et le cortex préfrontal dorsolatéral antérieur (aire de Brodmann 46) et pour la stimulation de ces zones du cerveau, comprenant au moins une électrode de stimulation (E) qui comporte au moins un contact capable d'acquérir un signal LFP ou qui est une électrode EEG à contact unique adaptée pour être montée de manière non invasive sur le cuir chevelu ou qui est une macroélectrode iEEG avec un contact adapté pour être monté dans le cortex cérébral ou sur sa surface, **caractérisé en ce que** le système comprend un système d'acquisition et de stimulation (US), un système de traitement (UP), un système d'analyse (UN), dans lequel l'électrode de stimulation (E) est connectée par fil ou sans fil et également de manière bidirectionnelle au système d'acquisition et de stimulation (US), qui est connecté au système de traitement (UP), qui est connecté au système d'analyse (UN), qui est reconnecté au système d'acquisition et de stimulation (US), et en outre, dans lequel le système d'acquisition et de stimulation (US) comprend un amplificateur (1), un convertisseur analogique-numérique (2), un tampon (3), un générateur (9) et un circuit de sélection de contact (12), tandis que l'amplificateur (1) est connecté au convertisseur analogique-numérique (2), qui est connecté au tampon (3), et le système de traitement (UP) comprend un système de filtrage (4), un système de mesure de la puissance spectrale et/ou du potentiel de champ (5) et un système de détection de défauts (6), tandis que le tampon (3) est connecté au système de filtrage (4) et que le système de filtrage (4) est connecté au système de détection de défauts (6) par l'intermédiaire du système de mesure de la puissance spectrale et/ou du potentiel de champ (5), dans lequel un procédé d'analyse spectrale de fréquence du signal est mis en œuvre, le système de mesure de la puissance spectrale et/ou du potentiel de champ (5) étant connecté à un système de seuil (7) situé dans le système d'analyse (UN), et le système de seuil (7) étant en outre connecté à un système de prise de décision (8) faisant partie du système d'analyse (UN), et le système de mesure de la puissance spectrale et/ou du potentiel de champ (5) et le système de seuil (7) sont connectés à un système d'optimisation (10) faisant partie du système d'analyse (UN) qui est connecté de manière bidirectionnelle au système de prise de décision (8), qui est connecté à un actionneur (9) dans le système d'acquisition et de stimulation (US), dans lequel l'actionneur (9) est connecté à l'amplificateur (1), et le circuit de décision (8) est connecté au circuit de sélection de contact (12), et le circuit de sélection de contact (12) est connecté à l'électrode de stimulation (E).

2. Système selon la revendication 1, dans lequel le système de filtrage (4) est connecté au système de mesure de la puissance spectrale et/ou du potentiel de champ (5).

3. Le système selon la revendication 1, dans lequel le système de mesure de la puissance spectrale et/ou du potentiel de champ (5) est connecté à un système de classification (11) situé dans le système d'analyse (UN), tandis que le système de classification (11) est connecté au système de prise de décision (8), et de plus, le système de mesure de la puissance spectrale et/ou du potentiel de champ (5) et le système de classification (11) sont connectés au système d'optimisation (10).

4. Système selon la revendication 3, dans lequel le système de mesure de la puissance spectrale et/ou du potentiel de champ (5) est connecté au système de détection de défauts (6) et au système de classification (11), tandis que le système de mesure de la puissance et/ou du potentiel de champ (5) est également connecté directement au système de classification (11).

5. Système selon la revendication 1, dans lequel le système de mesure de la puissance spectrale et/ou du potentiel de champ (5) est connecté au système de décision (8) par l'intermédiaire d'un système de classification (11).
